# EUROPEAN PATENT SPECIFICATION

(11) **EP 1 635 878 B1**
(45) Date of publication and mention of the grant of the patent: **29.12.2010**
(21) Application number: 04743873.4
(22) Date of filing: 17.06.2004
(51) Int. Cl.: A61K 49/08, A61K 49/14

(54) **PEPTIDE CONJUGATE FOR MAGNETIC RESONANCE IMAGING OF MATRIX METALLOPROTEINASES**
PEPTID-KONJUGAT FÜR DIE MAGNETRESONANZTOMOGRAPHIE VON MATRIX METALLOPROTEINASEN
CONJUGUES PEPTIDIQUES POUR IMAGERIE DES Métalloprotéinase matricielle PAR RESONANCE MAGNETIQUE

(30) Priority: 25.06.2003 FR 0307694; 25.09.2003 US 505423 P
(43) Date of publication of application: 22.03.2006
(73) Proprietor: GUERBET, 93420 Villepinte (FR)
(72) Inventor: PORT, Marc, F-95170 Deuil la Barre (FR); ROUSSEAUX, Olivier, F-60300 Senlis (FR); MEDINA, Christelle, F-77360 Vaires Sur Marne (FR); COROT, Claire, F-69006 Lyon (FR); GUILBERT, Irène, F-94400 Vitry Sur Seine (FR); RAYNAUD, Jean, Sébastien, F-92500 Rueil Malmaison (FR)
(74) Representative: Warcoin, Jacques
(86) International application number: PCT/IB2004/002210
(87) International publication number: WO 2004/112840

(56) References cited:
- EP-A- 0 345 359
- WO-A-01/60820
- WO-A1-98/47541
- WO-A2-2004/058275
- US-A1- 2002 098 149
- US-A1- 2002 122 806
- BREMER C ET AL: "IN VIVO MOLECULAR TARGET ASSESSMENT OF MATRIX METALLOPROTEINASE INHIBITION" NATURE MEDICINE, NATURE PUBLISHING, CO, US, vol. 7, no. 6, June 2001 (2001-06), pages 743-748, XP001164274 ISSN: 1078-8956
- PORTET D ET AL: "NONPOLYMERIC COATINGS OF IRON OXIDE COLLOIDS FOR BIOLOGICAL USE AS MAGNETIC RESONANCE IMAGING CONTRAST AGENTS" JOURNAL OF COLLOID AND INTERFACE SCIENCE, ACADEMIC PRESS, NEW YORK, NY, US, vol. 238, no. 1, 1 July 2001 (2001-07-01), pages 37-42, XP001162799 ISSN: 0021-9797
- LI W. P. ET AL: "Imaging matrix metalloproteinase expression in tumors" QUARTERLY JOURNAL OF NUCLEAR MEDICINE, vol. 47, 2003, pages 201-208, XP008041850
- GREENWALD R A: "Thirty-six years in the clinic without an MMP inhibitor. What hath collagenase wrought?" ANNALS OF THE NEW YORK ACADEMY OF SCIENCES 30 JUN 1999 LNKD- PUBMED:10415745, vol. 878, 30 June 1999 (1999-06-30), pages 413-419, ISSN: 0077-8923
- WEINREB: "In vivo imaging of matrix metalloproteinase activity with P947-enhanced MRI in atherosclerotic plaques of hypercholesterolemic rabbits" , 29 November 2006 (2006-11-29), Retrieved from the Internet: URL:http://rsna2006.rsna.org/rsna2006/v200 6/conference/event_display.cfm?em_id=44394 08>

## Description

The invention relates to new compounds and compositions for the imaging diagnostic of pathologies, namely for cardiovascular diseases, more precisely atherosclerosis disease. These compounds are contrast agents useful namely in the field of magnetic resonance imaging MRI, but also in other imaging fields such as nuclear medicine, X-ray, ultrasounds, optical imaging.

These compounds comprise at least a targeting moiety linked to at least a signal moiety.

A targeting entity is capable of targeting at least one marker of a pathologic state and/or area that are over or under expressed in a pathologic state and/or area compared to the non pathologic ones. These compounds are called specific compounds, the targeting entity being called biovector. Numerous signal entities/moieties are already known, such as linear or macrocyclic chelates of paramagnetic metal ion for MRI and of radionucleides for nuclear medicine. Such chelates are described in the documents EP 71 564, EP 448 191, WO 02/48119, US 6 399 043, WO 01/51095, EP 203 962 , EP 292 689 , EP 425 571 , EP 230 893 , EP 405 704, EP 290 047, US 6 123 920, EP 292 689, EP 230 893, US 6 403 055, WO 02/40060, US 6 458 337, US 6 264 914, US 6 221 334, WO 95/31444, US 5 573 752, US 5 358 704. Chelates commonly used are for example DTPA, DTPA BMA, DTPA BOPTA, DO3A, HPDO3A, TETA, DOTA (1,4,7,10-tetracyclododecane-N,N',N",N"'-tetraacetic acid), PCTA and their derivatives. Products on the market are namely for example Dotarem ® and Magnevist ®.The signal is measured in MRI by the relaxivity in water which is in the order of 3 to 10 mM-1s-1 Gd-1for such chelates.

There is still a serious need for a new contrast agent product able to target specifically atherosclerotic lesions.

Atherosclerosis is the most prevalent disease of modem society. A broad spectrum of clinically different diseases such as myocardial infarction, stroke, abdominal aneurysms and lower limb ischemia are basically related to atherosclerosis. Most of their acute manifestations share a common pathogenic feature : rupture of an atherosclerotic plaque with superimposed thrombosis. Plaque rupture, which accounts for approximately 70% of fatal acute myocardial infarctions and of symptomatic carotid lesions, is the ultimate complication of a vulnerable plaque. Vulnerable plaques include thrombosis-prone plaques as well as those with a high probability of undergoing rapid progression, thus becoming culprit plaques. They are characterized by a large lipid core, a thin cap and macrophage-dense inflammation on or beneath their surface. The risk of acute ischemic event for an individual is determined by the number of vulnerable plaques and the current challenge is to stratify such a risk.

Conventional imaging techniques are unable to detect and help characterize vulnerable plaques, especially those of the coronary arteries. Angiography is strictly an anatomic imaging tool and is unable to evaluate coronary plaque dimension and composition. Other modalities are catheter-based and, therefore, have a limited clinical applicability. Intravascular ultrasound provides some information on plaque morphology but image resolution and sensitivity are still insufficient to reliably distinguish vulnerable plaque deposits. Optical coherence tomography better delineates between intimal wall and plaque but its penetration depth is low. Angioscopy may be used to detect lipid-rich plaques and to visualize thrombus, whereas thermography is very sensitive to superficial inflammation. However, both techniques are unable to examine the deep layers of the arterial wall and to estimate cap thickness.

A very promising technique is the magnetic resonance imaging (MRI) technique. On atherosclerotic carotid plaques, it is able to visualize intraplaque hemorrhage and fibrous cap rupture, but also to detect intraluminal thrombi and differentiate their age. It is, therefore, a potentially attractive diagnostic tool for risk stratification of patients with recent onset of cerebral ischemic symptoms. Until now however, it lacks sufficient spatial resolution for accurate measurements of cap thickness and characterization of the coronary atherosclerotic lesions.

Thus for a predictive diagnostic there is a need for a physiological characterisation of the plaques further to their morphological study. An alternative strategy for identification, by MRI, of coronary vulnerable plaques may be to apply a molecular imaging approach based on the detection of a specific marker. One such marker is represented by the matrix metalloproteinases (MMPs), a family of zinc-containing endoproteinases which are overexpressed in active atherosclerotic lesions and promote plaque instability by degrading the fibrillar collagen of the fibrous cap. Thus, a contrast molecule, which can be detected namely with MRI, will be useful to image MMP activity and to non invasively detect vulnerable plaques and improve patients' risk stratification.

Although little is known about the amounts of MMPs accumulating within human vulnerable plaques, some studies have reported a surexpression of MMP-8 per milligram of tissue in advanced atherosclerotic carotid lesions. These levels were considered similar to those obtained for MMP-1 and MMP-13 . As the sensitivity of MRI in vivo is relatively low compared to scintigraphic imaging techniques, for instance, there is a need to compensate for the low levels of MMPs in the lesions in order to generate a sufficient signal intensity. This requirement may be achieved by using a compound which targets nonselectively the majority of MMPs and, thereby, will allow a high local concentration of the contrast agent. The applicant has now prepared imaging compounds comprising a biovector with good affinity for MMP-1, MMP-2, MMP-3, MMP-8, MMP-9 ; in particular MMP-3 are surexpressed in lesional plaques.

US 2002/0 122 806 discloses labelled peptides for MMP-detection.

Specific compounds for the targeting of MMPs are described in the prior art. For instance WO 01/60416 describes compounds that comprise a targeting entity towards MMPs coupled to a linear or macrocyclic chelate signal entity. According to applicant's knowledge based namely on biological assays, such compounds of the prior art are not sufficiently efficient for a very satisfying in vivo diagnosis, due to their relative low relaxivity which is in the order of 5 to 10 mMol-1s-1Gd-1 and/or their lack of affinity or selectivity. Thus there still remains a serious need for new products that are effectively efficient in imaging diagnostic in vivo

Surprisingly, while assessing very promising compounds with high relaxivity, the applicant has now shown that a particular peptidic MMP inhibitor coupled to a signal entity gives effective very good results for the diagnostic imaging despite the relatively low relaxivity of the signal entity. The compound prepared by the applicant is indeed very successful for the specific diagnosis of a disease associated with vulnerable plaques, compared to a non specific control compound Dotarem. The affinity of exemplified compounds for MMPs was tested in vitro on purified MMPs as well as ex vivo on WHHL rabbit arteries and human endarterectomy specimens. The biodistribution was studied in a mouse model of atherosclerosis showing increased MMP expression.

It is reminded here that a very high number of MMP targeting molecules are described in the prior art, which exhibit a high structural diversity, reviewed namely in :
- Current Medicinal Chemistry, 2001, 8, 425-474
- Chem rev, 1999, 99, 2735-2776
- DDT vol 1, n°1, jan 1996, Elsevier Science, 16-17
- Bioconjugate Chem, 2001, 12, 964-971

It is also reminded that over 150 US patents or patent applications cover MMP inhibitors and a lot more cover MMP targeting entities.

The peptidic MMP inhibitor used as biovector by the applicant is described in Biochemical and Biophysical research Communications, vol 199, 3, 1994, pages 1442-1446 and in US 5 100 874. But the coupling to a signal entity of this particular peptidic MMP inhibitor, among the huge amount of possible MMP targeting entities and inhibitors known with equivalent or higher affinity or selectivity for MMPs, was neither described nor suggested for diagnostic imaging and specially for cardiovascular disease diagnostic. Further, according to the applicant's knowledge, the clinical trials relating to MMP target entities in the therapeutic field focus on cancer therapy and are not engaged in the cardiovascular domain.

Thus according to a first aspect the invention relates to a diagnostic agent comprising a compound of formula (I)

(PEPTIDE) - (LINKER)n2 - (SIGNAL)

Wherein
1) PEPTIDE is :

   X1 - X2 - X3 - X4 - NHOH (II),

   wherein
   X1 is absent or X1 is a residue of an alpha-amino glycine and, X2 is a residue of an amino acid selected from proline, hydroxyproline, and thioproline, X3 is a residue of an amino acid selected from leucine and isoleucine and X4 is a residue of alanine;
   and the carboxyl group of alpha-amino acid X1 forms a peptide bond together with the amino group of alpha-amino acid X2, the carboxyl group of alpha-amino acid and acid X2 forms a peptide bond together with the amino group of alpha-amino acid X3, the carboxyl group of alpha-amino acid X3 forms a peptide bond together with the amino group of alpha-amino acid X4 and the carboxyl group of alpha- amino acid X4 forms an amido together with -NHOH ;
   and the hydrogen atom of the amino group in said alpha-amino acid X1 may be replaced with a member X0 selected from the group consisting of p-aminobenzoyl (ABz), p-amino-benzyl, p-hydroxybenzoyl (HBz), 3-p-hydroxyphenylpropionyl (HPP).
2) SIGNAL is a signal entity for medical imaging
3) LINKER eventually absent represents a chemical link between PEPTIDE and SIGNAL
4) n2 = 1 or 0
; and the pharmaceutical salts thereof.

The amino acids may be either D or L amino acids.

The term peptide functionally equivalent to peptide (II) refers to peptides that have a chemical structure allowing them to be coupled to the chelate, and a biological activity such that the activity of the diagnostic compound (I) is comparable to the activity of compound exemplified above towards MMPs, within the range of 20 to 200 %, typically at least 80% of the activity of exemplified compounds.

The activity towards MMPs may be equivalent towards each MMP targeted by the peptides or only towards certain of them ; thus the activity towards MMPs relates to the global MMPs targeting activity such that the compound is useful in term of medical imaging diagnostic of cardiovascular/atheroma diseases and in particular of vulnerable plaque detection.

In particular the peptide is preferably chosen so that the concentration which inhibits by 50% the activity of MMPs (IC50) is less than 10 µM. Preferably the IC50 is between 0.5 and 5 µM. However peptides with higher IC50 are also included if they give effectively good results in vivo imaging, in particular any peptide allowing to visualize the plaques in the ex vivo test exemplified in the detailed description.

According to the invention PEPTIDE is a peptide X1 - X2 -

X3-X4 NHOH (II)

wherein X1 is absent or X1 is glycine, X2 is a residue of an amino acid selected from proline, hydroxyproline, thioproline, X3 is a residue of an amino acid selected from leucine, isoleucine and X4 is a residue of alanine.

According to an embodiment PEPTIDE is a peptide

X1-X2-X3-X4-NHOH (II)

wherein X1 is glycine, X2 is proline, X3 is leucine, X4 is alanine. According to an embodiment PEPTIDE is X-NHOH with X chosen among : Abz-Gly-Pro-D-Leu-D-Ala, HBz-Gly-Pro-D-Leu-D-Ala, Abz-Gly-Pro-Leu-Ala, HPP-Pro-D-Leu-D-Ala, HPP-Pro-Leu-Ala.

In particular the PEPTIDE p-aminobenzoyl-Gly-Pro-D-Leu-D-Ala-NHOH is very satisfying for plaque imaging.

The hydroxamates peptides derivatives terminate NHOH, instead of peptides CO2H terminal.

The peptides can be carried out by processes which can be divided roughly into (A) and (B) below:
(A) Process where a compound of the formula Boc-X4 -NHOBzl is used as starting material; the peptide chain is extended on the Boc-N group side first to form the group X3 -X4 -, which is converted, via the group X2 -X3 -X4- into the group X1 -X2 -X3 -X4 -; and finally the O-benzyl on the hydroxamic acid side is eliminated to give the desired compound; and
(B) Process where a compound of the formula Boc-X4 -OR2 is used as starting material to synthesize the corresponding peptide derivative:
   X1 -X2 -X3 -X4 -OR2 (with R2 : methyl or ethyl group)
   Document US 5 100 874 describes precisely the preparation of such peptides, which is also available at BACHEM company (www.Bachem.com).

In the above mentioned processes, any means conventionally used in the peptide synthetic chemistry may be employed as specific means for condensing amino acids for formation of peptide chains ; for protecting with protecting groups the amino, imino, carboxyl and/or hydroxyl groups which may be present in their structure; and for eliminating such protecting groups.

Concerning the pharmaceutical salts :
- Prefered cations of inorganic bases suitable for salifying the complexes comprise, in particular, alkali or alkaline-earth metal ions such as potassium, sodium, calcium, magnesium.
- Preferred cations of organic bases comprise those of primary, secondary and tertiary amines, such as ethanolamine, diethanolamine, morpholine, glucamine, N-methylglucamine,N, N-dimethylglucamine.
- Preferred anions of inorganic acids comprise, in particular, the ions of halo acids such as chlorides, bromides, iodides or other ions such as sulfate.
- Preferred anions of organic acids comprise those of acids conventionally used in pharmaceutical technique for the salification of basic substances, such as acetate, succinate, citrate, fumarate, maleate, oxalate, trifluoroacetate

Preferred cations and anions of amino acids comprise, for example, those of taurine, glycine, lysine, arginine or ornithine or of the aspartic and glutamic acids.

According to an embodiment, SIGNAL is a linear or macrocyclic chelate. Chelates (chelators, chelating ligands) for magnetic resonance imaging contrast agents are selected to form stable complexes with paramagnetic metal ions, such as Gd (III), Dy (III), Fe (III), Mn (III) and Mn (II), include the residue of a polyaminopolycarboxylic acid, either linear or cyclic, in racemic or optically active form, such as ethylenediaminotetracetic acid (EDTA), diethylenetriaminopentaacetic acid (DTPA), N-[2-[bis(carboxymethyl)amino]-3-(4-ethoxyphenyl)propyl]-N-[2-[bis(carboxymethyl)amino]ethyl]-L-glycine (EOB-DTPA), N,N-bis[2-[bis(carboxymethyl)amino]ethyl]-L-glutamic acid (DTPA-GLU), N,N-bis[2-[bis(carboxymethyl)amino]ethyl]-L-lysine (DTPA-LYS), the DTPA mono- or bis-amide derivatives, such as N,N-bis[2-[carboxymethyl[(methylcarbamoyl)methyl]amino]ethyl] glycine (DTPA-BMA), 4-carboxy-5,8,11-tris(carboxymethyl)-1-phenyl-2-oxa-5,8,11-triazatridecan-13-oic acid (BOPTA), 1,4,7,10-tetraazacyclododecan-1,4,7,10-tetraacetic acid (DOTA), 1,4,7,10-tetraazacyclododecan-1,4,7-triacetic acid (DO3A), 10-(2-hydroxypropyl)-1,4,7,10-tetraazacyclododecan-1,4,7-triacetic acid (HPDO3A) 2-methyl-1,4,7,10-tetraazacyclododecan-1,4,7,10-tetraacetic acid (MCTA), ( alpha , alpha ', alpha ", alpha '")-tetramethyl-1,4,7,10-tetraazacyclododecan- 1,4,7,10-tetraacetic acid (DOTMA), 3,6,9,15-tetraazabicyclo[9.3.1]pentadeca-1(15),11,13-triene-3,6,9-triacetic acid (PCTA), or of a derivative thereof wherein one or more of the carboxylic groups are in the form of the corresponding salts, esters, or amides; or of a corresponding compound wherein one or more of the carboxylic groups is replaced by a phosphonic and/or phosphinic group, such as for instance 4-carboxy-5,11-bis(carboxymethyl)-1-phenyl-12-[(phenylmethoxy)methyl]-8-(phosphonomethyl)-2-oxa-5,8,11-triazatridecan-13-oic acid, N,N'-[(phosphonomethylimino)di-2,1-ethanediyl]bis[N-(carboxymethyl)glycine], N,N'-[(phosphonomethylimino)di-2,1-ethanediyl]bis[N-(phosphonomethyl)glycine], N,N'-[(phosphinomethylimino)di-2,1-ethanediyl]bis[N-(carboxymethyl)glycine], 1,4,7,10-tetraazacyclododecane-1,4,7,10-tetrakis[methylen(methylphosphonic)]acid, or 1,4,7,10-tetraazacyclododecane-1,4,7,10-tetrakis[methylen(methylphosphinic)]acid. Usable chelates may also be DOTA gadofluorins, DO3A, HPDO3A, TETA, TRITA, HETA, DOTA-NHS, M4DOTA, M4DO3A, PCTA and their derivatives, 2-benzyl-DOTA,alpha- (2-phenethyl) 1,4,7,10 tetraazacyclododecane-1-acetic-4,7,10-tris (methylacetic) acid, 2benzylcyclohexyldiethylenetriaminepentaacetic acid, 2-benzyl-6methyl-DTPA, and 6,6"-bis [N, N, N", N"tetra (carboxymethyl)aminomethyl)-4'- (3-amino-4-methoxyphenyl)- 2,2' : 6', 2"-terpyridine, N,N'-bis-(pyridoxal-5-phosphate) ethylenediamine- N, N'-diacetic acid (DPDP) and ethylenedinitrilotetrakis (methylphosphonic) acid (EDTP).

Suitable chelating ligands as well as the processes for their preparation are described for instance in the following patents : EP-A-230,893, EP-A-255,471, EP-A-299,795, EP-A-325,762, EP-A-565,930, EP-A-594,734, US-A-4,885,363, EP-A-773,936, WO-A-9426313, WO-A-9426754, WO-A-9426755, WO-A-9519347, WO-A-9731005, WO-A-9805625, WO-A-9805626, WO-A-9935133, WO-A-9935134, and WO-A-0146207.

Preferred chelating ligands are linear and macrocyclic polyaminopolycarboxylic acids, in racemic or optically active form.

Most preferred are (DTPA), (DOTA), (BOPTA), (DO3A), (HPDO3A), (DOTMA), (PCTA) and their derivatives.

The appropriate chelates are not limited to this list ; other chelates in compounds (I) with good efficiency in imaging diagnostic is appropriate. In particular SIGNAL may be of general formula or derivatives thereof described in detail in WO 01/60416 and US 6 221 334.

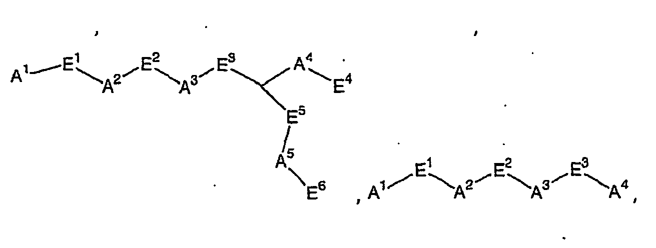

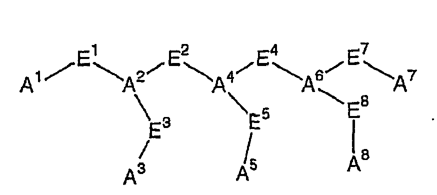

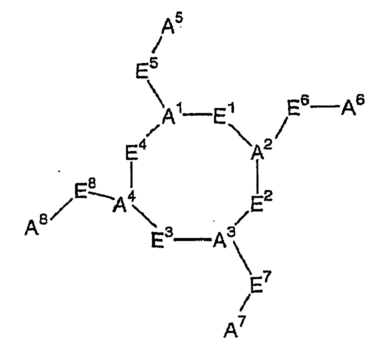

Preferred paramagnetic metal ions include ions of transition and lanthanide metals (i.e: metals having atomic number of 21 to 29,42, 43, 44, or 57 to 71). In particular ions of Mn, Fe, Co, Ni, Eu, Gd, Dy, Tm, and Yb are preferred, with those of Mn, Fe, Eu, Gd, and Dy being more preferred and Gd being the most preferred.

In nuclear medicine diagnostic, the metal chelate is selected to form stable complexes with the metal ion chosen for the particular application. Chelators or bonding moieties for diagnostic radiopharmaceuticals are selected to form stable complexes with the radioisotopes that have imageable gamma ray or positron emissions, such as 99Tc, 95Tc, 111In, 62Cu 60Cu 64Cu, 67Ga, 68Ga, 86Y.

Chelators for technetium, copper and gallium isotopes are selected preferably from diaminedithiols, monoamine-monoamidedithiols, triamide-monothiols, monoamine-diamide-monothiols, diaminedioximes, and hydrazines. The chelators are generally tetradentate with donor atoms selected from nitrogen, oxygen and sulfur. Preferred reagents are comprised of chelators having amine nitrogen and thiol sulfur, donor atoms and hydrazine bonding units. The thiol sulfur atoms and the hydrazines may bear a protecting group which can be displaced either prior to using the reagent to synthesize a radiopharmaceutical or preferably in situ during the synthesis of the radiopharmaceutical. Chelators for 111 In and 86Y are typically selected from cyclic and acyclic polyaminocarboxylates such as DTPA, DOTA,D03A, 2benzyl-DOTA, alpha-(2-phenethyl) 1,4,7,10-tetraazazcyclododecanel-acetic-4, 7,10-tris (methylacetic) acid, 2-benzylcyclohexyldiethylenetriaminepentaacetic acid, 2-benzyl-6-methyl

DTPA, and 6,6"-bis [N, N, N", N"-tetra (carboxymethyl) aminomethyl4'- (3-amino-4-methoxyphenyl)-2, 2' : 6', 2"-terpyridine.

In the above formula (I) PEPTIDE and SIGNAL can be bound together either directly (n2 = 0) or through a spacer LINKER. It is also possible to have, in the above formula (I), one MRI targeting entity (detectable moiety) bound to more than one peptide. In a realisation n2 =1. In another realisation (which is not part of the present invention), in order to increase the signal, several chelates are used ; example the linker is a divisor chemically linked to at least two chelates such as a 1,3,5 triazine divisor. When the MRI compounds of formula (I) contain more than one MRI detectable moiety (chelate), said detectable moieties can also be bound to more than one peptide through a spacer LINKER containing a multiplicity of binding sites.

For instance the LINKER consists of an alkylidene, alkenylidene, alkynylidene, cycloalkylidene, arylidene, or aralkylidene radical that can be substituted and be interrupted by heteroatoms such as oxygen, nitrogen, and sulphur. In a preferred embodiment said spacer arm consists of an aliphatic, straight or branched chain, that effectively separates the reactive moieties of the spacer so that ideally the spatial configuration of the molecule of the PEPTIDE is not influenced by the presence of the MRI detectable moiety and the PEPTIDE is thus more easily recognized by its MMP target. Said chain may be interrupted by groups such as, -O-, -S-, - CO-, -NR-, -CS- or by aromatic rings such as phenylene radicals, and may bear substituents such as -OR, -SR, -NRR1, - COOR, -CONRR1, wherein R and R1, each independently, may be a hydrogen atom or an organic group.

More globally, appropriate linker groups include, alkyl and aryl groups, including substituted alkyl and aryl groups and heteroalkyl (particularly oxo groups) and heteroaryl groups, including alkyl amine groups, as defined as follows.

Alkyl groups include straight or branched chain alkyl group, with straight chain alkyl groups being preferred. If branched, it may be branched at one or more positions, and unless specified, at any position. The alkyl group may range from about 1 to about 15 carbon atoms (C1-C15), with a preferred embodiment utilizing from about 1 to about 10 carbon atoms (C1-C10), with C1 to C5 being particularly preferred, although in some embodiments the alkyl group may be larger. Also included within the definition of an alkyl group are cycloalkyl groups such as C5 and C6 rings, and heterocyclic rings with nitrogen, oxygen, sulfur or phosphorus. Alkyl also includes heteroalkyl, with heteroatoms of sulfur, oxygen, nitrogen, and silicone being preferred. Alkyl includes substituted alkyl groups.

By "aryl group" or "aromati,c group" or grammatical equivalents herein is meant an aromatic monocyclic or polycyclic hydrocarbon moiety generally containing 5 to 14 carbon atoms (although larger polycyclic rings structures may be made) and any carbocylic ketone or thioketone derivative thereof, wherein the carbon atom with the free valence is a member of an aromatic ring. Aromatic groups include arylene groups and aromatic groups with more than two atoms removed. For the purposes of this application aromatic includes- heterocycle. "Heterocycle" or "heteroaryl" means an aromatic group wherein 1 to 5 of the indicated carbon atoms are replaced by a heteroatom chosen from nitrogen, oxygen, sulfur, phosphorus, boron and silicon wherein the atom with the free valence is a member of an aromatic ring, and any heterocyclic ketone and thioketone derivative thereof. Thus, heterocycle includes thienyl, furyl, pyrrolyl, pyrimidinyl, oxalyl, indolyl, purinyl, quinolyl, isoquinolyl, thiazolyl, imidozyl, etc. As for alkyl groups, the aryl group may be substituted with a substitution group.

Usable linker groups include p-aminobenzyl, substituted p-aminobenzyl, diphenyl and substituted diphenyl, alkyl furan such as benzylfuran, carboxy, and straight chain alkyl groups of 1 to 10 carbons in length. Preferred linkers include p-aminobenzyl, methyl, ethyl, propyl, butyl, pentyl, hexyl, acetic acid, propionic acid, aminobutyl, p-alkyl phenols, 4-alkylimidazole, carbonyls, OH, COOH, glycols such as PEG. By "methylene glycol" or "(poly)ethylene glycol" herein is meant a -(O-CH2-CH2)n- group, although each carbon atom of the ethylene group may also be singly or doubly substituted, i.e. -(O-CR2-CR2)n-, with R as described above. Ethylene glycol derivatives with other heteroatoms in place of oxygen (i.e. -(N-CH2-CH2)n- or -(S-CH2-CH2)n-, or with substitution- groups) are also appropriate. Squarate linkers are also usable.

According to another embodiment, SIGNAL is a carrier lipidic nanoparticle system capable of vehicling chelate signal entities. Several carrier systems are known, namely lipidic nanodroplets (emulsions of nanoparticles) such as described in WO 03/062198. This document describes the proof of concept of the specific imaging with nanodroplets carrying a biovector targeting alpha v beta 3 receptor. Other parent technologies may be used such as the ones described in US 6 403 056. One nanodroplet includes typically 10 000 to 100 000 chelates.

Many nanoparticulate emulsions may be used. For example W095/03829 describes oil emulsions where the drug is dispersed or solubilized inside an oil droplet and the oil droplet is targeted to a specific location by means of a ligand. US 5,542, 935 describes site-specific drug delivery using gas-filled perfluorocarbon microspheres. The targeting entity delivery is accomplished by permitting the microspheres to home to the target and then effecting their rupture. Low boiling perfluoro compounds are used to form the particles so that the gas bubbles can form. It is possible to employ emulsions wherein the nanoparticles are based on high boiling perfluorocarbon liquids such as those described in US 5,958, 371. The liquid emulsion contains nanoparticles comprised of relatively high boiling perfluorocarbons surrounded by a coating which is composed of a lipid and/or surfactant. The surrounding coating is able to couple directly to a targeting moiety or can entrap an intermediate component which is covalently coupled to the targeting moiety, optionally through a linker.

A possible emulsion is a nanoparticulate system containing a high boiling perfluorocarbon as a core and an outer coating that is a lipid/surfactant mixture which provides a vehicle for binding a multiplicity of copies of one or more desired components to the nanoparticle. The construction of the basic particles and the formation of emulsions containing them, regardless of the components bound to the outer surface is described in the above-cited patents to the present applicants, US 5 690 907, 5 780 010, 5 958 371.

Useful perfluorocarbon emulsions are reminded in US 6 676 963, and include those in which the perfluorocarbon compound is perfluorodecalin, perfluorooctane, perfluorodichlorooctane, perfluoro-n-octyl bromide, perfluoroheptane, perfluorodecane, perfluorocyclohexane, perfluoromorpholine, perfluorotripropylamine, perfluortributylamine, perfluorodimethylcyclohexane, perfluorotrimethylcyciohexane, perfluorodicyclohexyl ether, perfluoro-n-butyltetrahydrofuran, and compounds that are structurally similar to these compounds and are partially or fully halogenated (including at least some fluorine substituents) or partially or fully fluorinated including perfluoroalkylated ether, polyether or crown ether.

The lipid/surfactants used to form an outer coating on the nanoparticles (that will contain the coupled ligand or entrap reagents for binding desired components to the surface) include typically natural or synthetic phospholipids, fatty acids, cholesterols,lysolipids, sphingomyelins including lipid conjugated polyethylene glycol. Various commercial anionic, cationic, and nonionic surfactants can also be employed, including Tweens, Spans, Tritons. Some surfactants are themselves fluorinated, such as perfluorinated alkanoic acids such as perfluorohexanoic and perfluorooctanoic acids, perfluorinated alkyl sulfonamide, alkylene quaternary ammonium salts. In addition, perfluorinated alcohol phosphate esters can be employed. Cationic lipids included in the outer layer may be advantageous in entrapping ligands such as nucleic acids, in particular aptamers.

The lipid/surfactant coated nanoparticles are typically formed by microfluidizing a mixture of the fluorocarbon lipid which forms the core and the lipid/surfactant mixture which forms the outer layer in suspension in aqueous medium to form an emulsion. In this procedure, the lipid/surfactants may already be coupled to additional ligands when they are coated onto the nanoparticles, or may simply contain reactive groups for subsequent coupling. Alternatively, the components to be included in the lipid/surfactant layer may simply be solubilized in the layer by virtue of the solubility characteristics of the ancillary material. Sonication or other techniques may be required to obtain a suspension of the lipid/surfactant in the aqueous medium.

Typically, at least one of the materials in the lipid/surfactant outer layer comprises a linker or functional group which is useful to bind the additional desired component or the component may already be coupled to the material at the time the emulsion is prepared.

For coupling by covalently binding the targeting peptide or other organic moiety (such as a chelating agent for a paramagnetic metal) to the components of the outer layer, various types of bonds and linking agents may be employed. Typical methods for forming such coupling include formation of amides with the use of carbodiamides, or formation of sulfide linkages through the use of unsaturated components such as maleimide. Other coupling agents include, for example, glutaraldehyde, propanedial or butanedial, 2-iminothiolane hydrochloride, bifunctional N-hydroxysuccinimide esters such as disuccinimidyl suberate, disuccinimidyl tartrate, bis [2- (succinimidooxycarbonyloxy) ethyl] sulfone, heterobifunctional reagents such asN- (5-azido-2-nitrobenzoyloxy) succinimide, succinimidyl4- (N-maleimidomethyl) cyclohexane- 1-carboxylate, and succinimidyl 4- (p-maleimidophenyl) butyrate, homobifunctional reagents such as1, 5-difluoro-2,4-dinitrobenzene, 4, 4'-difluoro-3, 3'-dinitrodiphenylsulfone, 4,4'-diisothiocyano-2, 2'-disulfonic acid stilbene,p-phenylenediisothiocyanate, carbonylbis (L- methionine p-nitrophenyl ester), 4,4'-dithiobisphenylazide, erythritolbiscarbonate and bifunctional imidoesters such as dimethyl adipimidate hydrochloride, dimethyl suberimidate, dimethyl 3,3'-dithiobispropionimidate hydrochloride. Linkage can also be accomplished by acylation, sulfonation, reductive amination. A multiplicity of ways to couple, covalently, a desired ligand to one or more components of the outer layer is known in the art. The ligand itself may be included in the surfactant layer if its properties are suitable. For example, if the ligand contains a highly lipophilic portion, it may itself be embedded in the lipid/surfactant coating. Further, if the ligand is capable of direct adsorption to the coating, this too will effect its coupling.

Other useful encapsulation system may be used, if they exhibit sufficient hydrophily towards the PEPTIDE, for instance: mono or multilayers lipidic systems, liposomes, saccharidic polymers.

According to an other embodiment, SIGNAL is a metal nanoparticle based on iron such as ultra small superparamagnetic particles USPIO generally comprising an iron oxide or hydroxide. Preferably, such magnetic particles comprise a ferrite, especially maghemite (γ Fe₂O₃) and magnetite (Fe₃O₄), or also mixed ferrites of cobalt (Fe₂CoO₄) or of manganese (Fe₂MnO₄). Examples of suitably coated ferromagnetic or superparamagnetic particles are for instance those described in US patents 4,770,183, 4,827,945, 5,707,877, 6,123,920, and 6,207,134 having a coating materials, i.e., polymers such as polysaccharides, carbohydrates, polypeptides, organosilanes, proteins, gelatin-aminodextran, or starch and polyalkylene oxides, at the condition that they can be functionalised to allow binding of the particle to the spacer or directly to the PEPTIDE.

In a realisation, particles are coated with a phosphate, phosphonate, bisphosphonate or gem-bisphosphonate coating. A preferred gem-bisphosphonate coating is of formula X-L-CH (PO₃H₂)₂ where the biphosphontate part is linked to the particle and where:
X is a chemical function able to react with the PEPTIDE
L is an organic group linking X to the function gem-bisphosphonate -CH(PO₃H₂)₂ .

According to one particularly preferred embodiment, L represents a substituted or unsubstituted aliphatic group, and more preferably a group -(CH₂)ₚ-, where p is an integer from 1 to 5. According to another preferred embodiment, L represents a group L₁-CONH-L₂ and more preferably a group -(CH₂)ₙ-NHCO-(CH₂)ₘ- where n and m represent an integer from 0 to 5.

The X end of the gem-bisphosphonate compound of formula (I) is chosen in such a manner that it is capable of reacting and forming a covalent bond with a group present on the PEPTIDE biovector. For more information concerning these coupling processes reference may be made in particular to the work *Bioconjugate techniques,* Greg T. Hermanson, 1995, Publisher : Academic, San Diego, Calif.

There may be mentioned as preferred X groups, in particular:
- -COOH,
- -NH₂, -NCS, -NH-NH₂, -CHO, alkylpyrocarbonyl (-CO-O-CO-alk), acylazidyl (-CO-N₃), iminocarbonate (-O-C(NH)-NH₂), vinylsulphuryl (-S-CH=CH₂), pyridyldisulphuryl (-S-S-Py), haloacetyl, maleimidyl, dichlorotriazinyl, halogen,
- the groups -COOH and -NH₂ being especially preferred.

The applicant has also studied compounds which coating is a phosphonate or bisphosphonate or their derivatives other than the gem bisphosphonate.

According to another example which is not part of the present invention the SIGNAL entity is a fluorescent probe for fluorescent imaging. In near infrared fluorescence imaging, filtered light or a laser with a defined bandwidth is used as a source of excitation light. The excitation light travels through body tissues. When it encounters a near infrared fluorescent molecule ("contrast agent"), the excitation light is absorbed. The fluorescent molecule then emits light (fluorescence) spectrally distinguishable (slightly longer wavelength) from the excitation light.

In particular, fluorochromes destinated to the targeting of MMP have been described, for instance reminded in US2004015062. Enzyme-sensitive molecular probes have been synthesized and which are capable of fluorescence activation at 600-900 nm. These probes are described namely in US 6,083,486. Fluorescent probes (i.e., excitation at shorter wavelength and emission at longer wavelength) are ideally suited for studying biological phenomena, as has been done extensively in fluorescence microscopy. If fluorescent probes are to be used in living systems, the choice is generally limited to the near infrared spectrum (600-1000 nm) to maximize tissue penetration by minimizing absorption by physiologically abundant absorbers such as hemoglobin (<550 nm) or water (>1200 nm).

Typically the fluorochromes are designed to emit at 800+/-50 nm. A variety of NIRF molecules have been described and/or are commercially available, including: Cy5.5 (Amersham, Arlington Heights, III.); NIR-1 (Dojindo, Kumamoto, Japan); IRD382 (LI-COR, Lincoln, Nebr.); La Jolla Blue (Diatron, Miami, Fla.); ICG (Akorn, Lincolnshire, III.); and ICG derivatives (Serb Labs, Paris, France). Quantum dots derivatives (inorganic fluorophores comprising nanocristal) may also be used.

Another aspect of the present invention contemplates a method of imaging cardiovascular pathologies associated with extracellular matrix degradation, such as atherosclerosis, heart failure, and restenosis in a patient involving: (1) administering a paramagnetic metallopharmaceutical of the present invention capable of localizing the loci of the cardiovascular pathology to a patient by injection or infusion; and (2) imaging the patient using magnetic resonance imaging or planar CT or SPECT gamma scintigraphy, or positron emission tomography or sonography.

The invention also relates to a method for assessing vulnerable plaques combining a diagnostic imaging with a product of the invention and/or a morphologic analysis of the plaques and/or a study of stenoses.

The invention also relates to:
- A method of detecting, imaging or monitoring the presence of matrix MMPs in a patient comprising the steps of: a) administering to said patient a diagnostic agent described above; and b) acquiring an image of a site of concentration of said diagnostic agent in the patient by a diagnostic imaging technique.
- A method of detecting, imaging or monitoring a pathological disorder associated with MMPs activity in a patient comprising the steps of: a) administering to said patient a diagnostic agent described above ; and c) acquiring an image of a site of concentration of said diagnostic agent in the patient by a diagnostic imaging technique.

- A method above wherein the atherosclerosis is coronory atherosclerosis or cerebrovascular atherosclerosis.
- A method of identifying a patient at high risk for transient ischemic attacks or stroke by determining the degree of active atherosclerosis in a patient carrying out the method above.
- A method of identifying a patient at high risk for acute cardiac ischemia, myocardial infarction or cardiac death by determining the degree of active atherosclerosis by imaging the patient by the method above.

The MRI detectable species (I) according to the present invention may be administered to patients for imaging in an amount sufficient to give the desired contrast with the particular technique used in the MRI. Generally, dosages of from about 0.001 to about 5.0 mmoles of MRI detectable species (I) per kg of body weight are sufficient to obtain the desired contrast. For most MRI applications preferred dosages of imaging metal compound will be in the range of from 0.001 to 2.5 mmoles per kg of body weight.

The MRI detectable species (I) of the present invention can be employed for the manufacture of a contrast medium for use in a method of diagnosis by MRI involving administering said contrast medium to a human or animal being and generating an image of at least part of said human or animal being.

For said use the MRI detectable species (I) of the present invention may be formulated with conventional pharmaceutical aids, such as emulsifiers, stabilisers, antioxidant agents, osmolality adjusting agents, buffers, pH adjusting agents, and may be in a form suitable for parenteral administration, e.g. for infusion or injection.

Thus the MRI detectable species (I) according to the present invention may be in conventional administration forms such as solutions, suspensions, or dispersions in physiologically acceptable carriers media, such as water for injection.

Parenterally administrable forms, e.g. i.v. solutions, should be sterile and free from physiologically unacceptable agents, and have low osmolality to minimize irritation and other adverse effects upon administration. These parenterally administrable solutions can be prepared as customarily done with injectable solutions. They may contain additives, such as anti-oxidants, stabilizers, buffers, etc., which are compatible with the chemical structure of the MRI detectable species (I) and which will not interfere with the manufacture, storage and use thereof.

Further aspects of the invention will be described in the detailed description above.

### 1) Compounds based on gadolinium chelates: preparation and biological assays and imaging.

### Contrast medium

Compound B was obtained by grafting a human MMPs inhibitor of formula II p-aminobenzoyl-Gly-Pro-D-Leu-D-Ala-NHOH on Gd³⁺ chelator (DOTA) appropriately functionalised for coupling with an isothiocyanate linker , at a ratio 1:1.

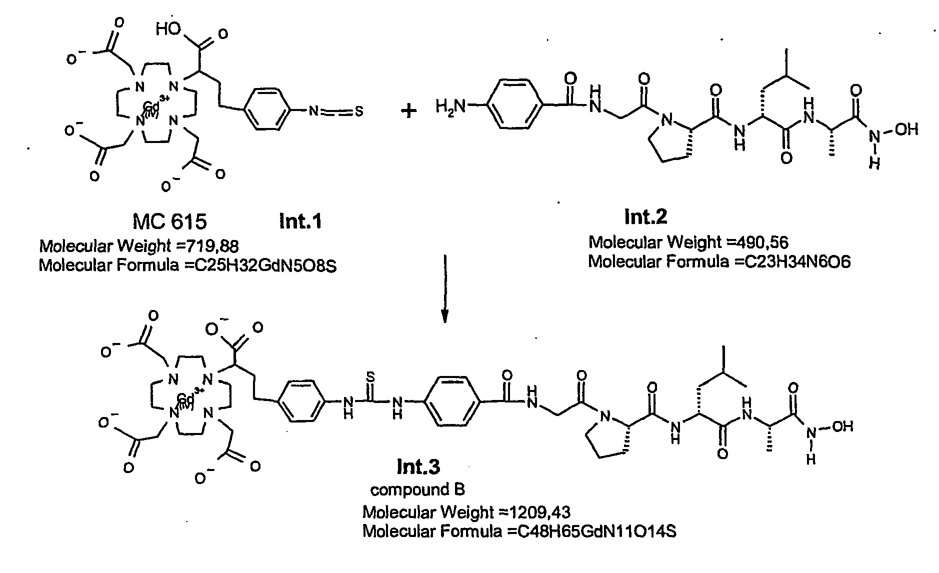

0.42g of Int1, 0.34g of peptide Abz-Gly-Pro-Leu-Ala-NHOH from Bachem and 200µL of triethylamine are dissolved in 20mL of DMSO at room temperature. The solution is stirred for 3 hours. 200mL of ether is instilled into the solution. The precipitate is washed with ether, ethanol and dichloromethane.
Analysis: HPLC purity = 92%; HPLC: Column: SYMMETRY C18, 5µm, 100Å, 250*4.6 mm

The chemical purity of this specific contrast medium was more than 90%. In addition, compound B displayed a molecular weight of 1210 Da and showed relaxivity values similar to that of gadoteric acid (Gd-DOTA, Dotarem©, GUERBET, France), a non specific product which was used as the reference compound. The MMP-inhibitor of compound B consisted of a water soluble tetrapeptidyl hydroxamic acid, purchased from Bachem (Budendorf, Switzerland).

### In vitro MMP inhibition assay

The ability of compound B or the corresponding peptide hydroxamate to inhibit MMP-1, MMP-2 or MMP-3 activity was tested *in vitro,* on human enzymes. The reaction was based on the formation of a fluorescent compound produced after MMP-cleavage of a substrate (see table 1 for experimental conditions). Briefly, compound B or the peptide alone was added at 3 different concentrations to a buffer containing the MMP to test. For the control sample, the contrast agent or the peptide was replaced by water. After a pre-incubation period of 30 min at 37°C, the fluorescence intensity was measured using a microplate reader (GeminiXS, Molecular Devices), in order to detect any compound interference with the fluorimetric assay (=F1). The enzymatic reaction was then initiated by the addition of the MMP substrate and the mixture was again incubated at 37°C. After a pre-determined time, a second fluorescent measurement was performed (=F2). The enzyme activity was determined by subtracting the signal F1 from F2, and the results were expressed as a percent inhibition of the control enzyme activity.
Moreover, the test was validated by using TIMP-1 or GM6001 as a standard inhibitor of MMPs see table 2).

### Biodistribution assay

*Animals -* the tissue distribution of compound B, when compared to Gd-DOTA, was performed in ApoE-KO mice in a 100% C57BI/6 background. The animals were bred and housed in the Animal Experiment Unit of the Center of Molecular and Vascular Biology, Leuven, Belgium, and had free access to food and water.
ApoE-KO mice of 11-12 weeks were fed a cholesterol-rich Western-type diet (Tecklad, Madison, USA) for 4 months. This model of atherosclerosis was fully characterized: it contained extensive atherosclerotic plaques in the heart and aortic arch, where the plaques occupied 34% of the total lumen area. Moreover, this animal model showed intense lipid staining, as well as the presence of macrophages, MMP-2 and MMP-3 which accounted for 15%, 10-20% and 20-40% of the plaque areas, respectively. *Biodistribution -* After the diet period, animals were matched for sex and body weight. compounfd B or Gd-DOTA was administered intravenously via the tail vein (n=7 / compound), at a clinical dose of 100 µmol Gd / kg. 60 min post-injection, the animals were anaesthetized and blood was obtained via eye bleeding. The mice were then subjected to a transcardiac perfusion with heparinized saline, in order to clear the intravascular compartment from any residual circulating contrast agent. Muscle and different organs (kidney, liver) were removed, as well as some artery specimens (aortic arch, carotid arteries, thoracic aorta, abdominal aorta and femoral artery). Finally, an aliquot of the blood sample was centrifuged and the Gd³⁺ contained in all the collected samples was quantified by induced coupled plasma - mass spectrometry (ICP-MS) after acidic mineralization.

### Ex vivo magnetic resonance imaging

*Specimen preparation -* Watanabe Heritable Hyperlipidemic rabbits (CAP, Olivet, France), which develop hypercholesterolemia and subsequent atherosclerosis due to a genetic deficiency of LDL receptors, were profoundly anaesthetized. After their sacrifice by exsanguination, the arterial tree, comprising the aortic arch with the beginning of the efferent arteries, as well as the thoracic aorta and the supra-renal abdominal aorta, was removed in one block and flushed with heparinized Krebs-Ringer Bicarbonate buffer (pH=7.4) in order to clean any remaining blood. The arterial specimen was then kept frozen at -20°C; it was gently and extemporarily unfrozen for experimental purpose.
Samples of fresh carotid artery were obtained from patients (n=21) undergoing carotid endarterectomy. Specimen consisted of the common carotid artery, as well as the internal and external carotid arteries. The excised plaques were rinsed in ice-cold saline and they were used in the 2-4 hours following surgery.
Finally, both rabbits and human atherosclerotic segments were cut in exactly 3.0 mm-thick sections, before their contact with the contrast medium and ex vivo imaging.
*Ex vivo incubation -* All the slices were deposited in a 24-wells culture plate. They were then incubated under stirring at 37°C, in 1.0 ml of Krebs-Ringer Bicarbonate buffer (pH = 7.4), Gd-DOTA or compound B (at a Gd concentration of 5 x 10⁻⁴ M). After pre-determined incubation periods of 1h, 3h or 18h, the slices originating from the WHHL rabbits were either immediately imaged, or subjected to a washing during 30 min, 1h or 3h (n=3 for each condition) in 10.0 ml of Krebs-Ringer bicarbonate buffer, with the medium being replaced every hour. Regarding human samples, only the protocol which allowed the best differentiation between the compounds (i.e. an incubation period of 18h, followed by a washing of 30 min) was applied. Afterwards, all the slices were drained and prepared for ex vivo imaging. Further the protocol was applied both regarding to lesional plaques and not lesional plaques specimens.
*Specificity testing -* To additionally test the specificity of the compound B uptake, we repeated the ex vivo 18h-incubation with a new set of WHHL rabbit atherosclerotic slices which were previously incubated during 30 min with the hydroxamate peptide, at a 10 times higher concentration than COMPOUND B (i.e. 5x10⁻³ M). The samples were subsequently washed during 30 min, as previously described.
*Detection of the contrast media by ex vivo imaging and Gd quantification -* For *ex vivo* MRI, the aortic slices were transferred in a new 24-wells plate and embedded in a semi-solid agar-agar gel (0.8% m/v) at room temperature. Images were performed on a 2.35 T MRI system (Biospec, Bruker, Germany), using a 7 cm inner diameter birdcage coil and a 200 mT/m insert gradient. A 3D T1w SNAP sequence was acquired with following parameters (TR/TE/a = 20 ms/4.2 ms/75°, Matrix 256*256*16, resolution 235*175*1000 µm³, duration 21'49") to have a compromise between resolution, short duration for screening and sections localization.

Finally, the gel was removed from the aortic segments and the Gd contained in each precisely weighed sample was quantified by ICP-MS after acidic mineralization.

*Immunohistochemistry -* In order to validate this ex vivo screening test and to correlate the MRI signal obtained for compound B with the targeted MMPs, we performed immunohistochemical analysis. The assays were conducted on incubated and washed sections originating from WHHL rabbits and humans. Regarding rabbits, only the antibodies recognizing MMP-2 (polyclonal antibody, Calbiochem, Darmstadt, Germany) and MMP-9 (monoclonal antibody, Oncogene Research Products, San Diego, USA) were commercially available and validated. For immunohistochemical analysis on human sections, the following antibodies were used for the detection of MMP-1 (rabbit anti-MMP-1 polyclonal antibody, Chemicon, Temecula, CA, USA), MMP-2 (rabbit anti-rat MMP-2 polyclonal antibody, Chemicon, Temecula, CA, USA), MMP-3 (rabbit anti-MMP-3 polyclonal antibody, Chemicon, Temecula, CA, USA), MMP-7 (rabbit anti-MMP-7 polyclonal antibody, Chemicon, Temecula, CA, USA) and MMP-9 (goat anti-MMP-9 polyclonal antibody, Santa Cruz Biotechnology, Santa Cruz, CA, USA). In both cases, the immunohistochemical analysis were performed according to manufacturer's instructions.

### Statistical analysis

Data were represented as means ± SEM. Sets of data were then compared with the unpaired non-parametric Mann-Whitney test, using a two-tail P-value (GraphPad Instat3). Differences were considered significant at P < 0.05.

### In vitro MMP inhibition assay

The results of the human MMP-inhibitory activity of compound B and its corresponding peptidyl hydroxamic acid are presented in table 2. Both compounds were effective *in vitro* on MMP-1 and 2, at a concentration of 1.0E-05 M, with compound B displaying an increased inhibitory activity when compared to the peptide. In contrast, no activity was noted for the two protease inhibitors on MMP-3. These experimental values matched perfectly with the reported IC50 values of the peptidyl hydroxamic acid on MMP-1 and -3, where the respective IC50 accounted for 1.0E-06 M and 1.5E-04 M. However, the results were slight stronger for MMP-2, as an enzymatic inhibition was already observed at a concentration of 1.0E-05 M for COMPOUND B and the peptide, whereas the IC50 was reported to be at 3.0E-05 M.

### Biodistribution assay

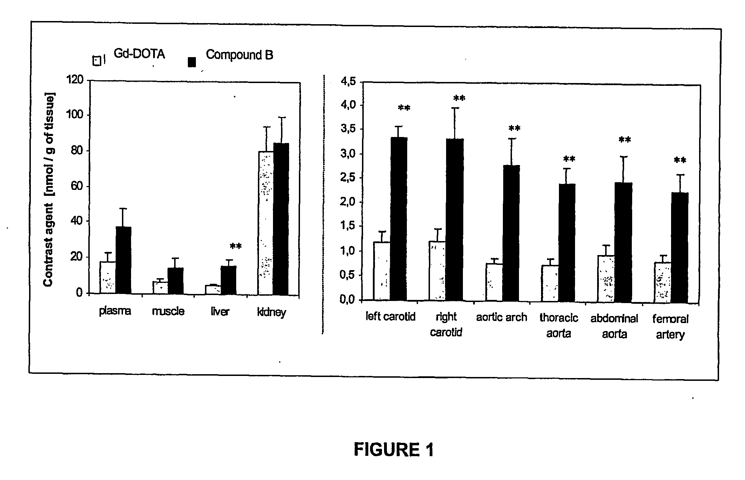

The biodistribution of compound B and its reference compound Gd-DOTA in the plasma, main organs and artery specimen of ApoE-KO mice is presented in figure 1. One hour post-intravenous injection, both contrast agents showed no significant difference in plasma, kidney and muscle accumulation, contrary to the liver uptake. In general, the concentrations of compound B and Dotarem© were poor in the main organs (i.e. < 1% ID), except in the kidney which is the organ of excretion. Moreover, the two contrast agents were cleared rapidly from the body, as the percent of injected dose found in the plasma was approximately 1-2% (half-life of elimination in plasma: 15 min).

Regarding the tissue distribution in the artery specimen, it revealed that compound B accumulated preferentially in the arterial wall, with a 3-3.5 times greater concentration when compared to Dotarem (p < 0.01). In addition, COMPOUND B, but not Dotarem©, showed a tendency to stain the arterial regions which contained the most numerous atherosclerotic plaques (carotid arteries and aortic arch).

### Ex vivo magnetic resonance imaging

*Assay performed on artery slices originating from Watanabe Heritable Hyperlipidemic rabbits -* After the 1 h-, 3h- or 18h- incubation periods, all the sections showed a strong, but diffuse, enhancement with the reference compound Gd-DOTA. This result was in agreement with the amount of Gd measured in the sections by ICP-MS, which attained 3-9 nmol / section (mean value). However, Gd-DOTA disappeared immediately during the first washing step of 30 min. At this moment, the Gd concentration dropped at a mean value inferior to 0.8 nmol / section.

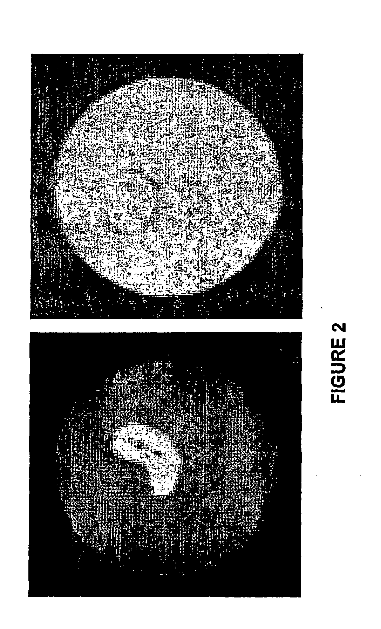

By contrast, compound B showed also a strong but more delineated enhancement after all the incubation periods (mean Gd concentration of 5-11 nmol / section), but this contrast agent was not eluted as fast as Gd-DOTA when the incubation period was longer than 1h. Indeed, when incubated during 3h or 18h, it still showed a clear signal after 30 min and 1h of washing, corresponding to a respective mean Gd concentration of 1-8 nmol / section (figure 2).

Moreover, when we performed an inhibition/competition study with a preliminary incubation with the free peptide, we founded a very faint enhancement only for compound B. This indicated that the inhibition / competition was effective and that compound B was specific for its target. Finally, this atherosclerotic model for ex vivo screening was validated, as it contained the MMPs of interest: the immunohistochemical analysis revealed a strong positivity for MMP-1 and a low expression of MMP-9.

*Assay performed on artery slices originating from humans undergoing surgical resection of the carotid artery -* As the previous experimental screening conditions (for example: 18h incubation, followed by 30 min washing) allowed to discriminate between the specific and the non specific contrast medium, and as both techniques of contrast medium detection, i.e. MRI ex vivo and Gd quantification by ICP-MS, correlated well, we applied them for the study on the human atheromatous sections. The results showed that only the specific compound B, and not the non specific compound Gd-DOTA, was able to enhance the atherosclerotic human sections, as demonstrated by MRI ex vivo and Gd quantification. In addition, the compound B allowed to differentiate between vulnerable plaques, which showed a strong positivity for MMP-1, -2, -3, -7 and -9 according to immunohistochemical analysis, and silent plaques, which contained a lower amount of MMPs. One can conclude that the compound B may be interesting for assessing the inflammatory degree, and hence the vulnerability, of human atherosclerotic plaques.

**Table 1: experimental conditions for the in vitro MMP-inhibition assay.**

| **Experimental procedures** | **MMP-1 [1]** | **MMP-2 [2]** | **MMP-3 [2]** |
|---|---|---|---|
| Enzyme concentration (activated enzyme) | 7 nM (enzyme isolated from human rheumatoid synovial fibroblast) | 0.35 µM (enzyme isolated from human rheumatoid synovial fibroblast) | 6 nM (recombinant enzyme expressed in sF9 cells) |
| Experimental medium | 50 mM Tris-HCl (pH=7.4), 200 mM NaCl, 5 mM CaCl₂, 0.02 mM ZnCl₂ and 0.05% Brij®35 | 50 mM Tris-HCl (pH=7.5), 150 mM NaCl, 10 mM CaCl₂, 0.02% NaN₃ and 0.05% Brij®35 | 25 mM Na acetate (pH=6.0), 150 mM NaCl, 10 mM CaCl₂, 0.02% NaN₃ and 0.05% Brij®35 |
| Substrate | 10 µM of DNP-Pro-Cha-Gly-Cys(Me)- His-Ala-Lys(n-Me- Abz)-NH₂ | 6 pM of Mca-Arg-Pro-Lys-Pro-Tyr-Ala-Nva-Trp-Met-Lys(DNP)-NH₂ (NFF-2) | 6 µM of Mca- Arg-Pro-Lys-Pro-Tyr-Ala-Nva-Trp-Met-Lys(DNP)-NH₂ (NFF-2) |
| Cleaved fluorescent | Cys(Me)-His-Ala-Lys(n-Me-Abz)-NH₂ | Mca-Arg-Pro-Lys-Pro-Tyr-Ala | Mca-Arg-Pro-Lys-Pro-Tyr-Ala |
| compound Fluorescent | λex = 360 nm | λex = 340 nm | λex = 340 nm |
| detection | λem = 465 nm | λem = 405 nm | λem = 405 nm |
| (wavelength) Reaction time | 40 min | 90 min | 90 min |

| | | | |
|---|---|---|---|
| [1] DA Bickett, MD. Green, J. Berman, M. Dezube, AS. Howe, PJ. Brown, JT Roth and GM McGeehan. A high throughput fluorigenic substrate for interstitial collagenase (MMP-1) and gelatinase (MMP-9). Anal. Biochem (1993) 212 : 58. [2] N. Nagase, CG. Fields and GB Fields. Design and characterization of a fluorogenic substrate selectively hydrolyzed by stromelysin 1 (MMP-3). J. Biol. Chem. (1994) 296: 20952. | | | |

**Table 2: In vitro effect of the protease inhibitors compound B and its corresponding peptide on human MMP-1,-2 and -3 activity, measured by a fluorimetric assay. Results were obtained in duplicate and are expressed as the mean of the percents of inhibition of the control value.**

| **MMP** | **Test concentration** | **Peptide** | **Compound B** |
|---|---|---|---|
| | **[M]** | **% inhibition of control values** | **% inhibition of control values** |
| MMP-1 | 1.0E-09 | 9 | 10 |
| MMP-1 | 1.0E-07 | 10 | 27 |
| MMP-1 | 1.0E-05 | 46 | 86 |
| MMP-2 | 1.0E-07 | 5 | 26 |
| MMP-2 | 1.0E-05 | 68 | 86 |
| MMP-2 | 1.0E-04 | 94 | 100 |
| MMP-3 | 1.0E-09 | 4 | 5 |
| MMP-3 | 1.0E-07 | 4 | 2 |
| MMP-3 | 1.0E-05 | 8 | 5 |
| Validation IC50 of TIMP on MMP-1 = 2.9E-09 M | | | |
| Validation IC50 of GM6001 on MMP-2 = 7.0E-10 M | | | |
| Validation IC50 of TIMP on MMP-3 = 4.8E-09 M | | | |

The figure 1 enclosed shows the biodistribution of compound B versus Gd-DOTA in plasma and main organs (left), as well as in the vascular wall of artery specimen (right) of ApoE-KO mice in a 100% C57BI/6 background (** : P < 0.01). The figure 2 shows the T1 MRI signal with compound B (left) compared to the control Dotarem (right).

### EXAMPLE 2) Compounds based on iron oxide superparamagnetic particles

### Sub example 2.8 : nitric acid ferrofluid (size PCS = 38 nm)

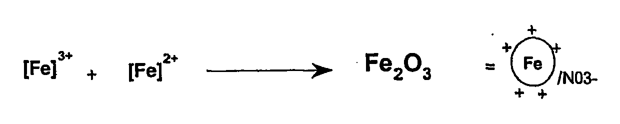

### Sub example 2.9 : nitric acid ferrofluid (size PCS =23,3 nm)

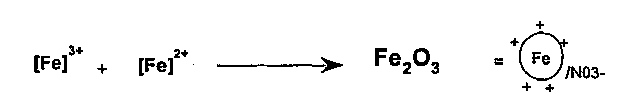

### Compound A

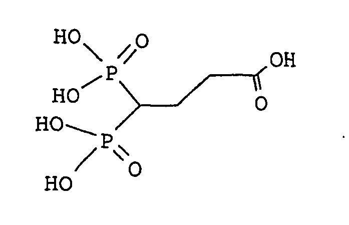

### Sub example 2.10 : (size PCS = 67,9 nm)

### Sub Ex 8 + compound A

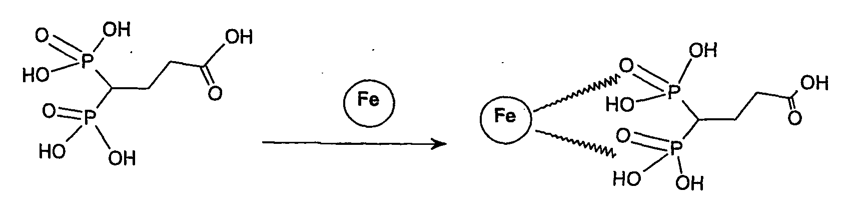

### Sub example 2. 11 : (size PCS = 40,3 nm)

### Sub Ex 8 + compound A + treatment pH 11

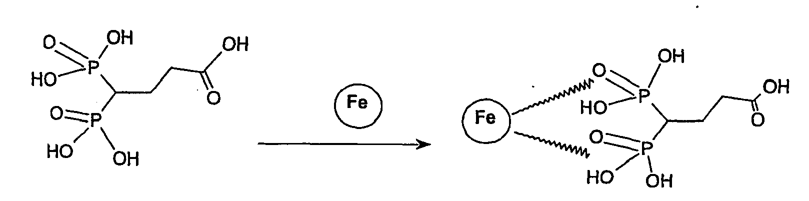

### Sub example 12 : (size PCS = 25,6 nm)

### Sub Ex 9 + compound A

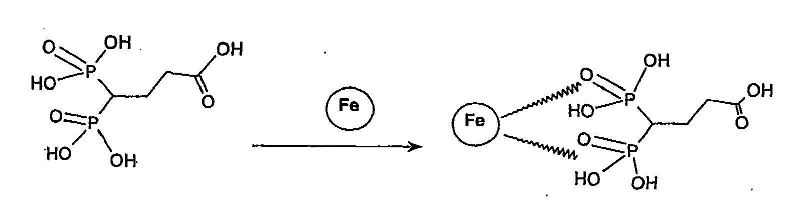

### Sub example 2.18 :

### Sub Ex 12 + compound PEPTIDE

### Preparation of compound A:

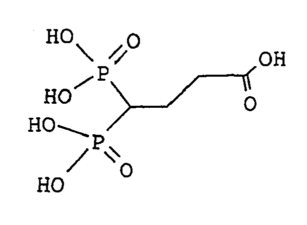

### 1) Diethyl-1-[ethoxyphosphoryl]vinyl phosphonate

13 g (0.433 mol) of paraformaldehyde and 10 ml (0.097 mol) of diethylamine are solubilized under hot conditions in 250 ml of methanol. 24 g (8.67 × 10⁻² mol) of diethyl[ethoxy(propyl)phosphoryl]methyl phosphonate are then added. The mixture is brought to reflux for 24 hours. The reaction medium is concentrated under vacuum. The concentrate is taken up twice with 250 ml of toluene and is then concentrated under vacuum.

The oil obtained is solubilized in 125 ml of toluene. 0.14 g of paratoluenesulfonic acid are added. The mixture is brought to reflux for 24 hours with a Dean-Stark trap and is then concentrated to dryness under vacuum.

The produdt is extracted with 500 ml of CH₂Cl₂ and is then washed twice with 250 ml of water. The organic phase is dried over MgSO₄ and concentrated under vacuum.

The crude product is purified on 625 g of Merck Geduran® silica gel (40-63 µm). Elution: CH₂Cl₂ /acetone - 50/50
18.4 g are isolated with a yield of 71 %.
Mass spectrum: M/z = 301.4 (ES+) theoretical M = 300.2
C¹³ spectrum: (s) 148.8 ppm, (t) 134.8-131.5 -128.2 ppm, (s) 62.2 ppm, (s) 16.7 ppm
H¹ spectrum: (t) 6.9 - 6.8 - 6.6 ppm, (unresolved peak) 3.9 ppm, (t) 1.15 ppm.

### 2) Diethyl 2-[2.2-bis(diethylphosphoryl)ethyl] malonate

1.6 g (0.01 mol) of diethyl malonate, 0.07 g (0.001 mol) of sodium ethoxide and 3 g (0.01 mol) of diethyl[ethoxy(propyl)phosphoryl]vinyl phosphonate are stirred for 15 min in 15 ml of ethanol. [TLC: SiO₂; eluent CH₂Cl₂ /acetone 50/50 - Rf = 0.6].

5 ml of a saturated NH₄Cl solution are added to the ethanolic solution. The mixture is concentrated under vacuum. The residue is extracted with 30 ml of ethyl acetate and washed twice with 5 ml of water. The organic phase is dried over MgSO₄ and is then evaporated to dryness.

The oil obtained is purified on 200 g of Merck Geduran^{®} silica
(40-63 µm). Elution CH₂Cl₂ /acetone 50/50
3.8 g are isolated with a yield of 82%.
Mass spectrum: M/z 460.9 (ES+), theoretical M = 460.

### 3) 4,4-Diphosphonobutanoic acid

7 g (15.7 × 10⁻² mol) of diethyl 2-[2.2-bis(diethylphosphoryl)ethyl] malonate are brought to reflux for 8 hours in 350 ml of HCl [5N].

The brown oil obtained is purified on 60 g of silanized silica 60 (0.063-0.200 mm) with water elution [HPLC monitoring].
3.6 g are isolated with a yield of 92%.
Mass spectrum: M/z 249 (ES+), theoretical M = 248
HPLC: column : Hypercarb^{®} 250 X 4 mm Detection: 202 nm
Isocratic eluent 99/1: 0.034 N H₂SO₄ / CH₃CN

### Sub example 2.8:

A solution of 36 g (0.181 mol) of FeCl₂.4H₂O and 20 ml of HCl at 37% in 150 ml of H₂O is introduced into a mixture consisting of 3 liters of water and 143 ml (0.302 mol) of FeCl₃ at 27%. 250 ml of NH₄OH at 25% are introduced rapidly with vigorous stirring. The mixture is stirred for 30 min. The liquors are removed by magnetic separation. The ferrofluid is washed 3 times consecutively with 2 liters of water.

The nitric ferrofluid is stirred for 15 min with 200 ml of HNO₃ [2M], and the supernatant is removed by magnetic separation.

The nitric ferrofluid is brough to reflux with 600 ml of water and 200 ml of Fe(NO₃)₃ [1 M] for 30 min. The supernatant is removed by magnetic separation.

The nitric ferrofluid is stirred for 15 min with 200 ml of HNO₃ [2M], the supernatant being removed by magnetic separation.

The nitric ferrofluid is washed 3 times with 3 liters of acetone, and is then taken up with 400 ml of water. The solution is evaporated under vacuum until a final volume of 250 ml is obtained.

| Concentration M/L | Z ave nm | Poly σ | Diameter SQUID | Ms emu/cm³ |
|---|---|---|---|---|
| 4.85 | 40 nm | 0.22 | 8.5 nm | 275 |

| | | | | |
|---|---|---|---|---|
| Ms (emu/cm³) = magnetization at saturation Z ave = Hydrodynamic size by PCS in unimodal mode Poly σ: peak polydispersity by PCS SQUID = diameter of the nongrafted particle (p) estimated by deconvolution of magnetization curves measured on a SQUID magnetometer | | | | |

### Sub example 2.9:

108 g (0.543 mol) of FeCl₂ . 4 H₂O in 450 ml of H₂O is introduced into a solution of 4 liters of water and 429 ml (0.906 mol) of FeCl₃ at 27%. 750 ml of NH₄OH at 25% are introduced rapidly with stirring (1200 rpm). The mixture is stirred for 30 min. The liquors are removed by magnetic separation. The ferrofluid is washed twice consecutively with 3 liters of water.

The nitric ferrofluid is stirred for ¼ H with 3 liters of HNO₃ [2M]; and the supernatant is removed by magnetic separation.

The nitric ferrofluid is brought to reflux with 1300 ml of water and 700 ml of Fe(NO₃)₃ [1 M] for 30 min (600 rpm). The supernatant is removed by magnetic separation.

The nitric ferrofluid is stirred for 15 min with 3 liters of HNO₃ [2M], the supernatant being removed by magnetic separation.

The nitric ferrofluid is washed 3 times with 3 liters of acetone, and is then taken up with 600 ml of water. The solution is evaporated under vacuum until a final volume of 250 ml is obtained.

### ❖ At this stage, the following characteristics are obtained:

| % yield | Concentration M/L | Z ave (nm) | Poly σ |
|---|---|---|---|
| 81.8 | 4.45 | 31.3 | 0.21 |

| | | | |
|---|---|---|---|
| Z ave = Hydrodynamic size by PCS in unimodal mode. | | | |

### ❖ Treatment:

200 ml of the preceding solution are stirred in 2,4 liters of HNO₃ for 4 hours. The supernatant is removed by magnetic separation. The nitric ferrofluid is washed twice with 3 liters of acetone, and is then taken up with 400 ml of water. The solution is evaporated under vacuum until a final volume of 250 ml is obtained.

| % yield | Concentration M/L | Z ave (nm) | Poly σ |
|---|---|---|---|
| 77 | 2.742 | 23.3 | 0.20 |

### Sub examples 2.10 to 2.12: Examples of complexation of the magnetic particles (p) by compounds gembisphosphonates

### Sub example 2.10:

50 ml of Sub example 2.8 at 4.85 M/L are diluted in 3 liters of water. A solution of 1.3 g (5.24 × 10⁻³ mol) of compound A from Example 1 in 100 ml of water is introduced dropwise. Stirring is maintained for 30 minutes. The flocculate is isolated by magnetic separation and is then washed 3 times with 3 liters of water.

It is redissolved with 700 ml of water at pH 7.2 with QS NaOH [1 N]. The final solution is filtered through a 0.22 µm membrane.

| |
|---|
| Iron titer = 0.252 M/L |
| Fe = 61.7 % mass/mass |
| P = 1.21 % mass/mass |
| C = 1.04 % mass/mass |
| Degree of grafting [compound A / Fe] = 1.86 % mol/mol |

### Sub example 2.11:

50 ml of Sub example 2.8 at 4.73 M/L are diluted in 3 liters of water. A solution of 1.3 g (5.24 × 10⁻³ mol) of compound A from Example 1 in 80 ml of water is introduced dropwise. Stirring is maintained for 30 minutes. The flocculate is isolated by magnetic separation and is then washed 3 times with 3 liters of water.

It is redissolved with 700 ml of water at pH 11 with QS NaOH [1 N] and then stabilized at pH 7.2 with QS HCl [1N]. The final solution is filtered through a 0.22 µm membrane.

| |
|---|
| Iron titer = 0.279 M/L |
| Fe = 63.9 % mass/mass |
| P = 1.38 % mass/mass |
| C = 1.07 % mass/mass |
| Degree of grafting [compound A / Fe] = 1.95% mol/mol |

### Sub example 2.12:

100 ml of example 2.9 at 2.742 M/L (PCS size = 21.3 nm) are diluted in 3 liters of water.

A solution of 1.5 g (6.03 × 10⁻³ mol) of compound A from example 1 in 100 ml of water is introduced dropwise. The stirring is maintained for 30 minutes. The floculate is isolated by magnetic separation and is then washed 3 times with 3 liters of water.

It is redissolved with 700 ml of water at pH 11 with QS NaOH [1 N] and then stabilized at pH 7.2 with QS HCl [1N]. The final solution is filtered through a 0.22 µm membrane.

| |
|---|
| Iron titer = 0.285 M/L |
| Fe = 62.9 % mass/mass |
| P = 1.32 % mass/mass |
| C = 1.22 % mass/mass |
| Degree of grafting [compound A / Fe] = 1.90% mol/mol |

### Relaxivities:

### 20MHz (0.47 T) - 37°C - in aqueous solution

| ri | r₂ |
|---|---|
| (mM⁻¹.s⁻¹) | (mM⁻¹.s⁻¹) |
| 35 ± 2 | 103 ± 5 |

### Example 2.18:

100 ml of a solution of Sub example 2.12 at 0.285 M/L are ultrafiltered through a PALL^{®} 30 KD stirring cell. 202 mg of compound PEPTIDE aminobenzoyl-Gly-Pro-D-Leu-D-Ala-NHOH from compound B are added to this solution. The pH is adjusted to 6.1 with QS HCl [0.1 N].

## Claims

1. Diagnostic agent comprising a compound of formula :
(PEPTIDE) - (LINKER)n2 - (SIGNAL) (I)
wherein
1) PEPTIDE is:
X1 - X2 - X3 - X4 - NHOH (II),
wherein
X1 is absent or X1 is a residue of an alpha-amino glycine, X2 is a residue of an amino acid selected from proline, hydroxyproline and thioproline , X3 is a residue of an amino acid selected from leucine and isoleucine and X4 is a residue of alanine
and the hydrogen atom of the amino group in said alpha-amino acid X1 may be replaced with a member X0 selected from the group consisting of p-aminobenzoyl (ABz), p-amino-benzyl, p-hydroxybenzoyl (HBz), 3-p-hydroxyphenylpropionyl (HPP),
2) SIGNAL is a signal entity for medical imaging chosen between macrocyclic or linear chelate and the derivatives thereof, a lipidic nanoparticle and an iron oxide particle,
3) LINKER eventually absent represents a chemical link between PEPTIDE and SIGNAL,
4) n2 = 1 ou 0
and the pharmaceutical salts thereof.

2. Diagnostic agent of claim 1 wherein PEPTIDE is X-NHOH with X chosen among : Abz-Gly-Pro-D-Leu-D-Ala, HBz-Gly-Pro-D-Leu-D-Ala, Abz-Gly-Pro-Leu-Ala, HPP-Pro-D-Leu-D-Ala, HPP-Pro-Leu-Ala.

3. Diagnostic agent of claims 1 or 2 wherein PEPTIDE is p-aminobenzoyl-Gly-Pro-D-Leu-D-Ala-NHOH.

4. Diagnostic agent of claims 1 to 3 wherein said agent is coupled to a metal element M chosen among an ion of a paramagnetic metal of atomic number 21-29, 42-44, or 58-70, namely Gd, or a radionucleide, typically ⁹⁹Tc, ¹¹⁷Sn, ¹¹¹In, ⁹⁷Ru, ⁶⁷Ga, ⁶⁸Ga, ⁸⁹Zr, ¹⁷⁷Lu, ⁴⁷Sc, ¹⁰⁵Rh; ¹⁸⁸Re, ⁶⁰Cu, ⁶²Cu, ⁶⁴Cu, ⁶⁷Cu, ⁹⁰Y, ¹⁵⁹Gd, ¹⁴⁹Pr, ¹⁶⁶Ho.

5. Diagnostic agent of claims 1 to 4 wherein SIGNAL is macrocyclic or linear chelate chosen among DTPA, DOTA, DTPA BMA, BOPTA, DO3A, HPDO3A, TETA, TRITA, HETA, M4DOTA, DOTMA, MCTA, PCTA, and the derivatives thereof.

6. Diagnostic agent of claims 1 to 4 wherein signal is an iron oxide particle coated with a gem-bisphosphonate.

7. Diagnostic agent of claims 1 to 6 wherein LINKER consists of an alkylidene, alkenylidene, alkylnylidene, cycloalkylidene, arylidene or aralkylidene radical that can be interrupted and be substituted by heteroatoms such as oxygen, nitrogen and sulphur.

8. Diagnostic agent of claim 7 wherein LINKER consists of an aliphatic straight or branched chain, which may be interrupted by groups such as -O-, -S-, -CO-, -NR-, -CS-, or by aromatic rings such as phenylene radicals, and may bear substituents such as -OR, -SR, -NRR1, -COOR, -CONRR1 wherein R and R1, each independently may be a hydrogen atom or an organic group.

9. Compound of claims 1 to 8 for use in the diagnostic of a cardiovascular/atheroma disease.

10. Use of compound of claims 1 to 8 for the preparation of an agent for the diagnostic of a cardiovascular/atheroma disease.

11. Method of preparation of a compound of claims 1 to 8 comprising the coupling of a peptide X1 -X2 -X3 -X4-NHOH and a SIGNAL entity.

12. Method of detecting, or imaging the presence of matrix metalloproteinase in a patient comprising the steps of: a) administering to said patient a diagnostic agent according to claims 1 to 8, b) acquiring an image of a site of concentration of said diagnostic agent in the patient by a diagnostic imaging technique.

13. Compound of claims 1 to 8 for use in detecting, imaging or monitoring atherosclerosis, heart failure, restenosis, transient cerebral ischemic attacks or stroke, acute cardiac ischemia, myocardial infarction or cardiac death in a patient by the administration of the compound to said patient and the acquisition of an image of a site of concentration of said compound in the patient by a diagnostic imaging technique.

## Patentansprüche

1. Diagnostikum, enthaltend eine Verbindung der Formel:
(PEPTID) - (LINKER)n2 - (SIGNAL) (I)
wobei
1) PEPTID für:
X1 - X2 - X3 - X4 - NHOH (II)
steht, wobei
X1 fehlt oder X1 für einen alpha-Aminoglycinrest steht, X2 für einen Rest einer Aminosäure ausgewählt aus Prolin, Hydroxyprolin und Thioprolin steht, X3 für einen Rest einer Aminosäure ausgewählt aus Leucin und Isoleucin steht und X4 für einen Alaninrest steht
und das Wasserstoffatom der Aminogruppe in der alpha-Aminosäure X1 durch ein Glied X0 ausgewählt aus der aus p-Aminobenzoyl (ABz), p-Aminobenzyl, p-Hydroxybenzoyl (HBz) und 3-p-Hydroxyphenylpropionyl (HPP) bestehenden Gruppe ersetzt sein kann,
2) SIGNAL für eine Signaleinheit für die medizinische Bilddarstellung ausgewählt aus makrocyclischen und geradkettigen Chelaten und den Derivaten davon, einem lipidischen Nanopartikel und einem Eisenoxidpartikel steht,
3) LINKER gegebenenfalls fehlt und für ein chemisches Bindeglied zwischen PEPTID und SIGNAL steht,
4) n2 = 1 oder 0
oder ein pharmazeutisches Salz davon.

2. Diagnostikum nach Anspruch 1, wobei PEPTID für X-NHOH steht, wobei X ausgewählt ist aus: Abz-Gly-Pro-D-Leu-D-Ala, HBz-Gly-Pro-D-Leu-D-Ala, Abz-Gly-Pro-Leu-Ala, HPP-Pro-D-Leu-D-Ala, HPP-Pro-Leu-Ala.

3. Diagnostikum nach Anspruch 1 oder 2, wobei PEPTID für p-Aminobenzoyl-Gly-Pro-D-Leu-D-Ala-NHOH steht.

4. Diagnostikum nach einem der Ansprüche 1 bis 3, wobei das Mittel an ein Metallelement M gekoppelt ist, das aus den Ionen eines paramagnetischen Metalls der Atomnummer 21-29, 42-44 oder 58-70, nämlich Gd, oder eines Radionukleids, typischerweise ⁹⁹Tc, ¹¹⁷Sn, ¹¹¹In, ⁹⁷Ru, ⁶⁷Ga, ⁶⁸Ga, ⁸⁹Zr, ¹⁷⁷Lu, ⁴⁷Sc, ¹⁰⁵Rh, ¹⁸⁸Re, ⁶⁰Cu, ⁶²Cu, ⁶⁴Cu, ⁶⁷Cu, ⁹⁰Y, ¹⁵⁹Gd, ¹⁴⁹Pr, ¹⁶⁶Ho ausgewählt ist.

5. Diagnostikum nach einem der Ansprüche 1 bis 4, wobei SIGNAL für ein makrocyclisches oder geradkettiges Chelat ausgewählt aus DTPA, DOTA, DTPA, BMA, BOPTA, DO3A, HPD03A, TETA, TRITA, HETA, M4DOTA, DOTMA, MCTA, PCTA und den Derivaten davon steht.

6. Diagnostikum nach einem der Ansprüche 1 bis 4, wobei SIGNAL für ein mit einem gem-Bisphosphonat beschichtetes Eisenoxidpartikel steht.

7. Diagnostikum nach einem der Ansprüche 1 bis 6, wobei LINKER aus einem Alkyliden-, Alkenyliden-, Alkylnyliden-, Cycloalkyliden-, Aryliden- oder Aralkylidenrest besteht, der durch Heteroatome wie Sauerstoff, Stickstoff und Schwefel substituiert und unterbrochen sein kann.

8. Diagnostikum nach Anspruch 7, wobei LINKER aus einer aliphatischen geradkettigen oder verzweigten Kette besteht, die durch Gruppen wie -O-, -S-, -CO-, -NR-, -CS- oder durch aromatische Ringe wie Phenylenreste unterbrochen sein kann und Substituenten wie -OR, -SR, -NRR1, -COOR, -CONRR1, wobei R und R1 jeweils unabhängig voneinander für ein Wasserstoffatom oder eine organische Gruppe stehen, tragen kann.

9. Verbindung nach einem der Ansprüche 1 bis 8 zur Verwendung bei der Diagnose einer Herzkreislauferkrankung/ einer atheromatösen Erkrankung.

10. Verwendung einer Verbindung nach einem der Ansprüche 1 bis 8 zur Herstellung eines Mittels zur Diagnose einer Herzkreislauferkrankung/ einer atheromatösen Erkrankung.

11. Verfahren zur Herstellung einer Verbindung nach einem der Ansprüche 1 bis 8, bei dem man ein Peptid X1 - X2 - X3 - X4 - NHOH und eine SIGNALEinheit kuppelt.

12. Verfahren zum Nachweis oder zur bildlichen Darstellung des Vorhandenseins von Matrixmetalloproteinase in einem Patienten, bei dem man: a) dem Patienten ein Diagnostikum nach einem der Ansprüche 1 bis 8 verabreicht, b) durch ein diagnostisches bildgebendes Verfahren ein Bild einer Anreicherungsstelle des Diagnostikums in dem Patienten akquiriert.

13. Verbindungen nach einem der Ansprüche 1 bis 8 zur Verwendung beim Nachweis, der bildlichen Darstellung bzw. der Kontrolle von Atherosklerose, Herzinsuffizienz, Restenose, transienten zerebralen ischämischen Anfällen oder Schlaganfall, akuter kardialer Ischämie, Herzinfarkt oder Herztod bei einem Patienten durch Verabreichung der Verbindung an den Patienten und die Akquisition einer Abbildung einer Anreicherungsstelle der Verbindung in dem Patienten durch ein diagnostisches bildgebendes Verfahren.

## Revendications

1. Agent diagnostique comprenant un composé de formule
(PEPTIDE) - (LIEUR) n2- (SIGNAL) (I)
dans laquelle
1) PEPTIDE est :
X1 - X2 - X3 - X4 - NHOH (II)
où
X1 est absent, ou X1 est un résidu d'une alpha-aminoglycine, X2 est un résidu d'un acide aminé choisi parmi la proline, l'hydroxyproline et la thioproline, X3 est un résidu d'un acide aminé choisi parmi la leucine et l'isoleucine, et X4 est un résidu d'alanine,
et l'atome d'hydrogène du groupe amino dudit acide alpha-aminé X1 peut être remplacé par un membre X0 choisi dans le groupe consistant en p-aminobenzoyle (ABz), p-amino-benzyle, p-hydroxybenzoyle (HBz), 3-p-hydroxyphénylpropionyle (HPP),
2) SIGNAL est une entité signal pour imagerie médicale, choisie parmi un chélate macrocyclique ou linéaire et les dérivés de celui-ci, une nanoparticule lipidique et une particule d'oxyde de fer,
3) LIEUR, éventuellement absent, représente un lien chimique entre PEPTIDE et SIGNAL,
4) n2 = 1 ou 0,
et ses sels pharmaceutiques.

2. Agent diagnostique de la revendication 1, dans lequel PEPTIDE est X-NHOH, X étant choisi parmi Abz-Gly-Pro-D-Leu-D-Ala, HBz-Gly-Pro-D-Leu-D-Ala, Abz-Gly-Pro-Leu-Ala, HPP-Pro-D-Leu-D-Ala, HPP-Pro-Leu-Ala.

3. Agent diagnostique de la revendication 1 ou 2, dans lequel PEPTIDE est p-aminobenzoyl-Gly-Pro-D-Leu-D-Ala-NHOH.

4. Agent diagnostique des revendications 1 à 3, dans lequel ledit agent est couplé à un élément métallique M choisi parmi un ion d'un métal paramagnétique ayant un numéro atomique 21-29, 42-44 ou 58-70, plus précisément Gd, ou un radionucléide, typiquement ⁹⁹TC, ¹¹⁷Sn, ¹¹¹In, ⁹⁷Ru, ⁶⁷Ga, ⁶⁸Ga, ⁸⁹Zr, ¹⁷⁷Lu, ⁴⁷Sc, ¹⁰⁵Rh ; ¹⁸⁸Re, ⁶⁰Cu, ⁶²Cu, ⁶⁴Cu, ⁶⁷Cu, ⁹⁰Y, ¹⁵⁹Gd, ¹⁴⁹Pr, ¹⁶⁶Ho.

5. Agent diagnostique des revendications 1 à 4, dans lequel SIGNAL est un chélate macrocyclique ou linéaire choisi parmi DTPA, DOTA, DTPA BMA, BOPTA, D03A, HPD03A, TETA, TRITA, HETA, M4DOTA, DOTMA, MCTA, PCTA et les dérivés de ceux-ci.

6. Agent diagnostique des revendications 1 à 4, dans lequel SIGNAL est une particule d'un oxyde de fer revêtue d'un gem-bisphosphonate.

7. Agent diagnostique des revendications 1 à 6, dans lequel LIEUR consiste en un radical alkylidène, alcénylidène, alkynylidène, cycloalkylidène, arylidène ou aralkylidène, qui peut être interrompu et substitué par des hétéroatomes tels que l'oxygène, l'azote et le soufre.

8. Agent diagnostique de la revendication 7, dans lequel LIEUR consiste en une chaîne aliphatique droite ou ramifiée, qui peut être interrompue par des groupes tels que -O-, -S-, -CO-, -NR-, -CS- ou par des noyaux aromatiques tels que des radicaux phénylène, et peut porter des substituants tels que -OR, -SR, -NRR1, -COOR, -CONRR1 où R et R1 peuvent, chacun indépendamment de l'autre, être un atome d'hydrogène ou un groupe organique.

9. Composé des revendications 1 à 8 pour utilisation dans le diagnostic d'une maladie cardiovasculaire/athéromateuse.

10. Utilisation du composé des revendications 1 à 8 pour la préparation d'un agent destiné au diagnostic d'une maladie cardiovasculaire/athéromateuse.

11. Procédé de préparation d'un composé des revendications 1 à 8, comprenant le couplage d'un peptide X1-X2-X3-X4-NHOH et d'une entité SIGNAL.

12. Procédé de détection ou d'imagerie de la présence d'une métalloprotéinase de matrice chez un patient, comprenant les étapes de : a) administration audit patient d'un agent diagnostique selon les revendications 1 à 8, b) acquisition d'une image d'un site de concentration dudit agent diagnostique chez le patient par une technique d'imagerie diagnostique.

13. Composé des revendications 1 à 8 pour utilisation dans la détection, l'imagerie ou la surveillance de l'athérosclérose, de l'insuffisance cardiaque, de la resténose, des attaques ischémiques cérébrales transitoires ou des accidents vasculaires cérébraux, de l'ischémie cardiaque aiguë, d'un infarctus du myocarde ou la mort cardiaque chez un patient, par administration du composé audit patient et acquisition d'une image d'un site de concentration dudit composé chez le patient par une technique d'imagerie diagnostique.
